# EUROPEAN PATENT APPLICATION

(11) **EP 4 190 903 A1**
(43) Date of publication of application: **07.06.2023**
(21) Application number: 21849153.8
(22) Date of filing: 27.07.2021
(51) Int. Cl.: C12N 15/61, C12N 9/90

(54) **ENZYME AGENT FOR USE IN EPIMERIZATION REACTION CATALYST FOR SUGAR, METHOD FOR PRODUCING EPIMERIZATION REACTION PRODUCT, AND EPIMERIZATION REACTION PRODUCT**

(30) Priority: 31.07.2020 JP 2020130365
(71) Applicant: Nihon Shokuhin Kako Co., Ltd., Tokyo 100-0005 (JP)
(72) Inventor: KANAI Mioka, Fuji-shi, Shizuoka 417-8530 (JP); IIZUKA Takahisa, Fuji-shi, Shizuoka 417-8530 (JP); KANAI Kenta, Fuji-shi, Shizuoka 417-8530 (JP)
(74) Representative: Michalski Hüttermann & Partner Patentanwälte mbB
(86) International application number: PCT/JP2021/027632
(87) International publication number: WO 2022/025022

(57) **Abstract**

An object of the present invention is to provide an enzyme preparation that efficiently catalyzes the epimerization reaction with little byproducts. The present invention provides an enzyme preparation for catalyzing an epimerization reaction of a saccharide, which contains any of the proteins (a) to (c) mentioned below, and wherein the saccharide is any selected from the group consisting of glucose, mannose, talose and galactose: (a) a protein consisting of the amino acid sequence of SEQ ID NO: 1 or 3; (b) a protein consisting of an amino acid sequence having an amino acid sequence identity of 90% or higher to the amino acid sequence of SEQ ID NO: 1 or 3, and having an activity for catalyzing an epimerization reaction of a saccharide; and (c) a protein consisting of an amino acid sequence derived from the amino acid sequence of SEQ ID NO: 1 or 3 by substitution, insertion, deletion and/or addition of one or several amino acids, and having an activity for catalyzing an epimerization reaction of a saccharide.

## Description

### Technical Field

### Cross-reference of related application

This application claims convention priority based on Japanese Patent Application No. 2020-130365 filed in Japan on July 31, 2020, of which entire description is herein incorporated by reference.

The present invention relates to an enzyme preparation for catalyzing an epimerization reaction of a saccharide, a method for producing an epimerization reaction product comprising the step of allowing said enzyme preparation to act to obtain an epimerization reaction product, and an epimerization reaction product obtained by said method.

### Background Technique

Mannose is an isomer of glucose, and is well known as a constituent saccharide of glucomannan, which is extracted from konjak etc., and hemicellulose, or of glycans present on cell surfaces etc. It is also known that mannose itself is contained in cranberries.

The bioavailability of mannose is low, and most of orally ingested mannose is quickly excreted in urine. It is believed that causative bacteria of urinary tract infections adhere to the surface of the urinary tract through binding of mannose-binding lectins of such bacteria to the saccharide chains present on the surface cells of the urinary tract and cause the infections. Since orally ingested mannose is quickly excreted in urine, it is expected that the excreted mannose has an effect of preventing urinary tract infection based on inhibition of the binding of the mannose-binding lectins of the bacteria and cell surface saccharide chains (Non-patent document 1).

Patent document 1 discloses a technique relating to an inhibitor of infection by harmful bacteria or feed for preventing infection by harmful bacteria, which utilize a saccharide composition containing mannose as an active ingredient. Patent document 2 describes a food texture modifier for foods and beverages utilizing mannose as an active ingredient.

Several chemical reaction methods and enzymatic methods are known as preparation methods of mannose. Each of these methods will be discussed below.

The first chemical reaction method is a method for preparing mannose by reducing sucrose to produce sorbitol and mannitol, separating mannitol, and then oxidizing mannitol. However, this method has problems concerning efficiency because it requires multiple steps and catalytic reactions at high temperatures and high pressures.

The second chemical reaction method is a method for preparing mannose by hydrolyzing glucomannan or mannan. However, this method has problems that the substrate is expensive, and the preparation of the substrate is labor intensive.

The third chemical reaction method is a method utilizing hydrolysis of hemicellulose of copra meal or the like. However, this method has problems that the yield of mannose is poor due to the variety of constituent saccharides, and in the case of utilizing acid hydrolysis, the yield is even worse, and the purification load is large.

The fourth chemical reaction method is a method for preparing mannose by isomerizing glucose under alkaline conditions. However, this method has problems of low yield and high purification load due to decomposition of saccharides.

The fifth chemical reaction method is a method for preparing mannose by similarly isomerizing fructose under alkaline conditions. However, this method has problems in terms of yield and purification load, like the fourth chemical reaction method, and also has problems in that fructose is relatively expensive compared with glucose, and fructose remains in the reaction system, making it difficult to separate it from mannose.

The first enzymatic method is a method for preparing mannose by allowing a mannose isomerase to act on fructose. However, it has problems in terms of the price of fructose as the raw material and separation of residual fructose in the reaction system.

The second enzymatic method is a method for preparing mannose by allowing an aldose-ketose isomerase (hereinafter referred to as "AKI") to act on glucose or fructose. However, this method also has a problem in terms of separation of residual fructose in the reaction system.

For saccharides of which reducing terminal saccharide residues consist of two or more saccharide residues linked through the β-1,4 bond, such as cellobiose, cellobiose 2-epimerase (hereinafter referred to as "CE") is known as an enzyme that catalyzes the 2-epimerization (isomerization) reaction of the hydroxyl group at the 2-position of the reducing terminal saccharide residues or the reverse reaction thereof (Patent documents 3 and 4). This enzyme can catalyze the conversion of cellobiose, in which glucose is β-1,4-linked, to 4-O-β-glucosylmannose, or the reverse reaction thereof. In addition, Patent document 4 describes that mannose was obtained by allowing CE to act on glucose (third enzyme method). However, in the third enzymatic method using CE described in Patent document 4, CE also catalyzes the aldose-ketose isomerization reaction in addition to the epimerization reaction. Therefore, fructose is produced along with mannose, and thus the method has a problem in terms of separation of the by-produced fructose.

The fourth enzymatic method is a method using an enzyme that catalyzes a reaction of epimerizing the hydroxyl group at the 2-position of glucose, mannose, galactose, or talose (Patent documents 5 and 6). This method is superior to the first to third enzymatic methods in that almost no fructose and tagatose is produced as a byproduct (Patent documents 5 and 6).

### Prior Art References

### Patent documents

Patent document 1: Japanese Patent No. 4694667
Patent document 2: Japanese Patent Unexamined Publication (Kokai) No. 2001-275583
Patent document 3: Japanese Patent No. 5092049
Patent document 4: International Publication WO2010/090095
Patent document 5: Japanese Patent Unexamined Publication (Kokai) No. 2019-033702
Patent document 6: Japanese Patent No. 6657453

### Non-patent document

Non-patent document 1: Sharma V, Ichikawa M, Freeze HH, Mannose metabolism: more than meets the eye, Biochem. Biophys. Res. Commun., 2014;453(2):220-228, doi:10.1016/j.bbrc.2014.06.021

The entire descriptions of Patent documents 1 to 6 and Non-patent document 1 are incorporated herein by reference.

### Summary of the Invention

### Object to be achieved by the invention

The production efficiency of epimerization reaction products in the enzymatic methods depends largely on the characteristics of the enzyme used. There has been a need for enzyme preparations that more efficiently catalyze epimerization reactions of substrates, which are suitable for use on an industrial scale. Furthermore, they also have a problem that the byproducts reduce production efficiency not only by reducing the yield of the target reaction product, but also requiring effort for separation.

An object of the present invention is to provide an enzyme preparation that efficiently catalyzes the epimerization reaction with little byproducts.

### Means for achieving the object

The inventors of the present invention have analyzed expressions and properties of genes encoding various proteins for the purpose of searching for mannose epimerases. During such analysis, they focused on the gene present in the genome of *Lunatimonas lonarensis* and encoding a putative N-acylglucosamine 2-epimerase protein (GenBank: WP_010853281.1), and analyzed the expression thereof. As a result, they found that the protein has a 2-epimerization activity of catalyzing mutual conversions between mannose and glucose, and galactose and talose.

The inventors of the present invention also elucidated that mannose can be efficiently produced by using that protein, and accomplished the present invention.

The present invention is embodied as follows.
[1] An enzyme preparation for catalyzing an epimerization reaction of a saccharide, which contains any of the proteins (a) to (c) mentioned below, and wherein the saccharide is any selected from the group consisting of glucose, mannose, talose and galactose:
   (a) a protein consisting of the amino acid sequence of SEQ ID NO: 1 or 3;
   (b) a protein consisting of an amino acid sequence having an amino acid sequence identity of 90% or higher to the amino acid sequence of SEQ ID NO: 1 or 3, and having an activity for catalyzing an epimerization reaction of a saccharide; and
   (c) a protein consisting of an amino acid sequence derived from the amino acid sequence of SEQ ID NO: 1 or 3 by substitution, insertion, deletion and/or addition of one or several amino acids, and having an activity for catalyzing an epimerization reaction of a saccharide.
[2] An enzyme preparation for catalyzing an epimerization reaction of a saccharide, which contains a culture supernatant of a transformed cell into which any of the polynucleotides (d) to (f) mentioned below has been introduced, and wherein the saccharide is any selected from the group consisting of glucose, mannose, talose and galactose:
   (d) a polynucleotide consisting of the nucleotide sequence of SEQ ID NO: 2 or 4;
   (e) a polynucleotide that hybridizes with a complementary strand of the polynucleotide consisting of the nucleotide sequence of SEQ ID NO: 2 or 4 under stringent conditions, and encodes a protein having an activity for catalyzing an epimerization reaction of a saccharide; and
   (f) a polynucleotide having a nucleotide sequence identity of 90% or higher to the polynucleotide consisting of the nucleotide sequence of SEQ ID NO: 2 or 4, and encoding a protein having an activity for catalyzing an epimerization reaction of a saccharide.
[3] A method for producing an epimerization reaction product of a saccharide, which comprises the step of allowing the enzyme preparation according to [1] or [2] to act on a saccharide substrate to obtain the epimerization reaction product of a saccharide.
[4] A method for producing a food, feed, feed for fish culture, cosmetic, or medicament, which comprises the step of obtaining the food, feed, feed for fish culture, cosmetic, or medicament by using an epimerization reaction product of a saccharide obtained by implementing the method according to [3].

### Effect of the Invention

According to the present invention, a novel enzyme preparation having an epimerization activity can be provided.

### Brief Description of the Drawings

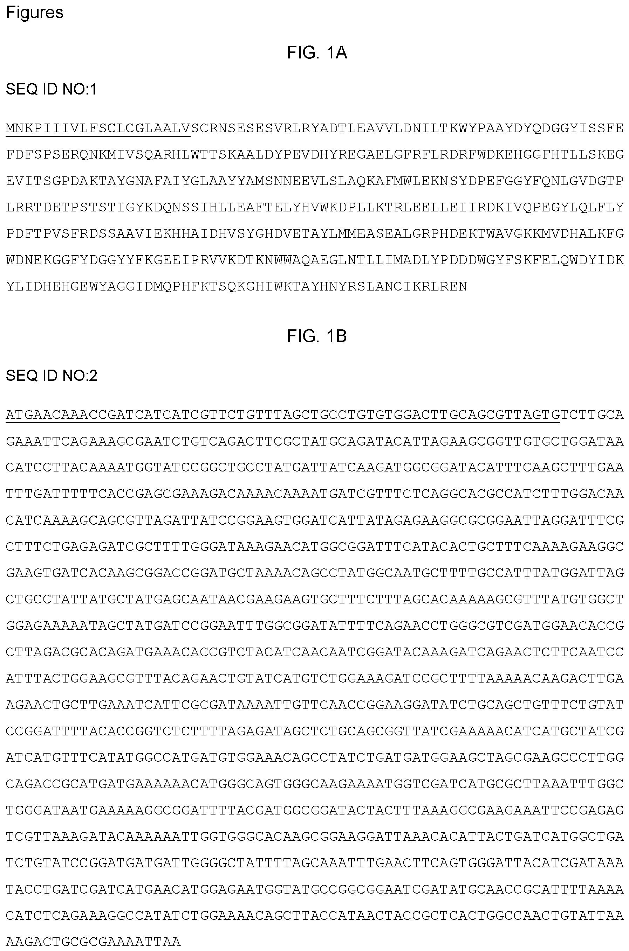

[Fig. 1A] Fig. 1A shows the amino acid sequence of the hypothetical protein derived from *Lunatimonas lonarensis* (SEQ ID NO: 1). The underlined region is a part deduced to be a signal peptide sequence.
[Fig. 1B] Fig. 1B shows the nucleotide sequence encoding the amino acid sequence of the hypothetical protein derived from *Lunatimonas lonarensis* (SEQ ID NO: 2). The underlined region is a nucleotide sequence corresponding to the sequence encoding the signal peptide sequence. The nucleotide sequence of SEQ ID NO: 4 is a sequence obtained from the nucleotide sequence of SEQ ID NO: 2 by deleting the nucleotide sequence corresponding to the sequence encoding the signal peptide sequence.

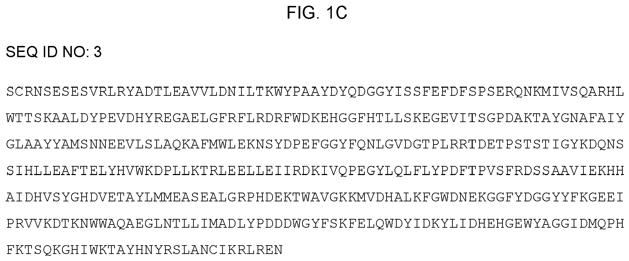

[Fig. 1C] Fig. 1C shows the amino acid sequence of the hypothetical protein derived from *Lunatimonas lonarensis* (SEQ ID NO: 3). The amino acid sequence of SEQ ID NO: 3 is a sequence obtained from the amino acid sequence of SEQ ID NO: 1 by deleting the part deduced to be a signal peptide.

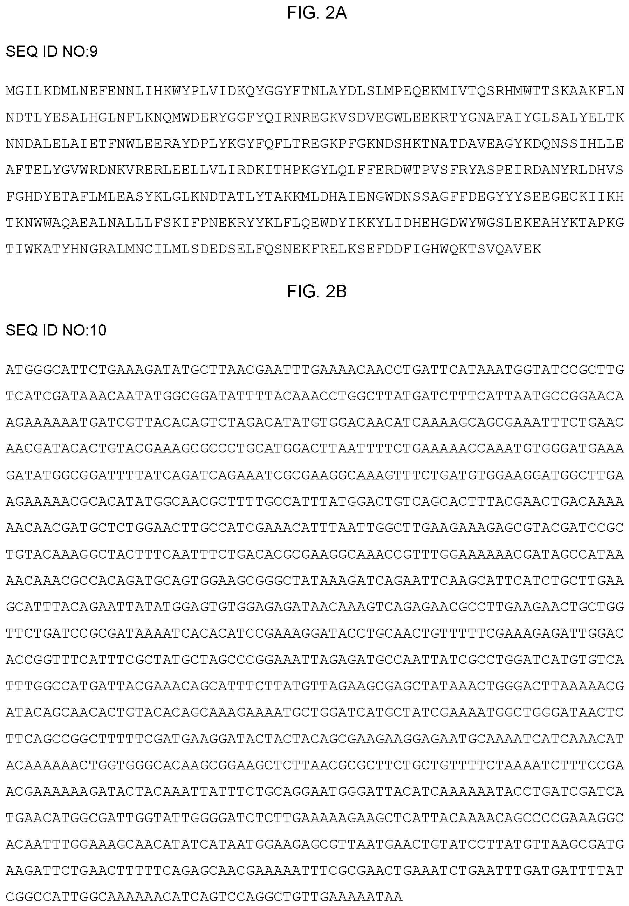

[Fig. 2A] Fig. 2A shows the amino acid sequence of the protein derived from *Melioribacter roseus* (SEQ ID NO: 9) used as a comparative example.
[Fig. 2B] Fig. 2B shows the nucleotide sequence encoding the amino acid sequence of the protein derived from *Melioribacter roseus used* as a comparative example (SEQ ID NO: 10).

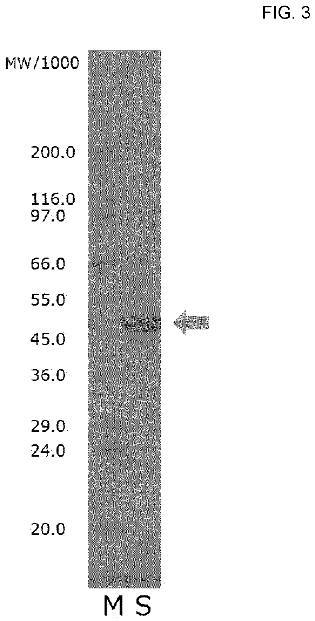

[Fig. 3] Fig. 3 shows results of SDS-PAGE analysis of the protein derived from *Lunatimonas lonarensis* obtained by the culture in Example 1 (obtained by filtration of the culture supernatant through a filter). The band of the protein derived from *Lunatimonas lonarensis* is indicated with an arrow. M, marker; and S, sample.

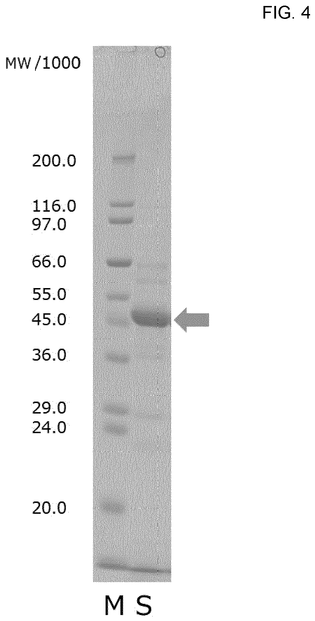

[Fig. 4] Fig. 4 shows results of SDS-PAGE analysis of the protein derived from *Lunatimonas lonarensis* obtained by the culture in Example 2 (obtained by filtration of the culture supernatant through a filter). The band of the protein derived from *Luna timonas lonarensis is* indicated with an arrow. M, marker; and S, sample.

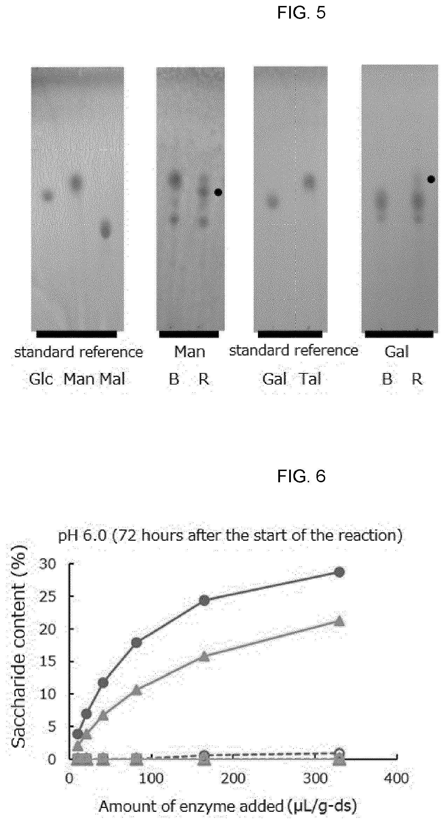

[Fig. 5] Fig. 5 shows results of TLC analysis of the enzyme reaction product. The meanings of the abbreviations are as follows: Glc, glucose; Man, mannose; Mal, maltose; Gal, galactose; Tal, talose; B, before enzyme reaction; and R, after enzyme reaction. The black circles (●) indicate the reaction product.
[Fig. 6] Fig. 6 is a graph showing the mannose and fructose contents in the reaction products obtained by allowing the solution 1 of the enzyme of the present invention, comparative enzyme solution, and empty vector control to act on glucose for 72 hours under a condition of pH 6.0. The amounts of the enzymes added were 10.3, 20.6, 41.3, 82.5, 165.0 and 330.0 µL/g-ds. The mannose contents and fructose contents were calculated from the peak area ratios obtained in HPLC analysis. ●, Solution 1 of the enzyme of the present invention (mannose content); ∘, solution 1 of the enzyme of the present invention (fructose content); ▲, comparative enzyme solution (mannose content); Δ, comparative enzyme solution (fructose content); ■, empty vector control (mannose content), and □: empty vector control (fructose content).

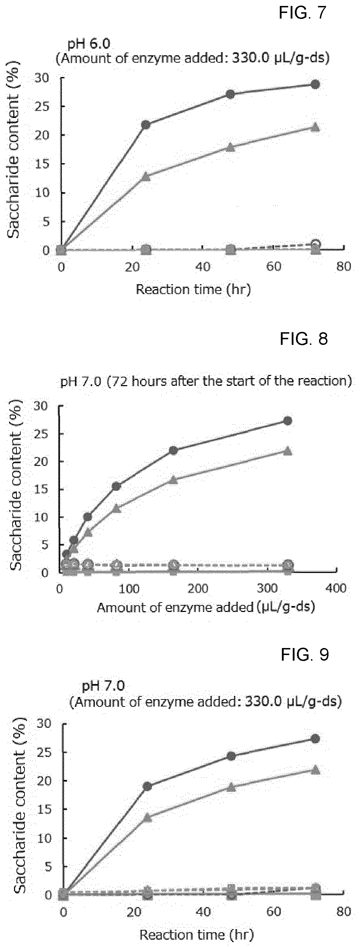

[Fig. 7] Fig. 7 is a graph showing the changes over time of the mannose and fructose contents in the reaction products obtained by allowing the solution 1 of the enzyme of the present invention, comparative enzyme solution, and empty vector control to act on glucose under a condition of pH 6.0. The amounts of the enzymes added were 330.0 µL/g-ds. The mannose contents and fructose contents were calculated from the peak area ratios obtained in HPLC analysis. ●, Solution 1 of the enzyme of the present invention (mannose content); ∘, solution 1 of the enzyme of the present invention (fructose content); ▲, comparative enzyme solution (mannose content); Δ, comparative enzyme solution (fructose content); ■, empty vector control (mannose content), and □: empty vector control (fructose content).
[Fig. 8] Fig. 8 is a graph showing the mannose and fructose contents in the reaction products obtained by allowing the solution 1 of the enzyme of the present invention, comparative enzyme solution, and empty vector control to act on glucose for 72 hours under a condition of pH 7.0. The amounts of the enzymes added were 10.3, 20.6, 41.3, 82.5, 165.0 and 330.0 µL/g-ds. The mannose contents and fructose contents were calculated from the peak area ratios obtained in HPLC analysis. ●, Solution 1 of the enzyme of the present invention (mannose content); ∘, solution 1 of the enzyme of the present invention (fructose content); A, comparative enzyme solution (mannose content); Δ, comparative enzyme solution (fructose content); ■, empty vector control (mannose content), and □: empty vector control (fructose content).
[Fig. 9] Fig. 9 is a graph showing the changes over time of the mannose and fructose contents in the reaction products obtained by allowing the solution 1 of the enzyme of the present invention, comparative enzyme solution, and empty vector control to act on glucose under a condition of pH 7.0. The amounts of the enzymes added were 330.0 µL/g-ds. The mannose contents and fructose contents were calculated from the peak area ratios obtained in HPLC analysis. ●, Solution 1 of the enzyme of the present invention (mannose content); ∘, solution 1 of the enzyme of the present invention (fructose content); ▲, comparative enzyme solution (mannose content); Δ, comparative enzyme solution (fructose content); ■, empty vector control (mannose content), and □: empty vector control (fructose content).

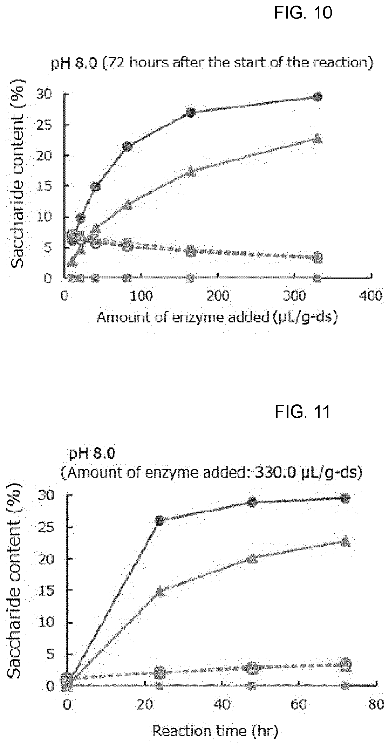

[Fig. 10] Fig. 10 is a graph showing the mannose and fructose contents in the reaction products obtained by allowing the solution 1 of the enzyme of the present invention, comparative enzyme solution, and empty vector control to act on glucose for 72 hours under a condition of pH 8.0. The amounts of the enzymes added were 10.3, 20.6, 41.3, 82.5, 165.0 and 330.0 µL/g-ds. The mannose contents and fructose contents were calculated from the peak area ratios obtained in HPLC analysis. ●, Solution 1 of the enzyme of the present invention (mannose content); ∘, solution 1 of the enzyme of the present invention (fructose content); A, comparative enzyme solution (mannose content); Δ, comparative enzyme solution (fructose content); ■, empty vector control (mannose content), and □: empty vector control (fructose content).
[Fig. 11] Fig. 11 is a graph showing the changes over time of the mannose and fructose contents in the reaction products obtained by allowing the solution 1 of the enzyme of the present invention, comparative enzyme solution, and empty vector control to act on glucose under a condition of pH 8.0. The amounts of the enzymes added were 330.0 µL/g-ds. The mannose contents and fructose contents were calculated from the peak area ratios obtained in HPLC analysis. ●, Solution 1 of the enzyme of the present invention (mannose content); ∘, solution 1 of the enzyme of the present invention (fructose content); ▲, comparative enzyme solution (mannose content); Δ, comparative enzyme solution (fructose content); ■, empty vector control (mannose content), and □: empty vector control (fructose content).

### Modes for Carrying out the Invention

The explanations of the present invention described below may be made with reference to representative embodiments or specific examples thereof, but the present invention is not limited to such embodiments. In this description, the mentioned saccharides are D-isomers, unless especially noted. The numerical ranges expressed by using "to" means a range including the numerical values described before and after "to" as the minimum and maximum values. In this description, values indicated with the term "about" are intended to mean numerical ranges of the values ± 10%.

As for the saccharide compositions (%) of the reaction products produced with the enzyme preparation of the present invention, the ratio of each saccharide was calculated as the area ratio (%) of the peak corresponding to each saccharide to the total area of the peaks detected by HPLC, which was taken as 100.

### (Enzyme preparation)

The present invention provides an enzyme preparation for catalyzing an epimerization reaction of a saccharide, which contains any of the proteins (a) to (c) mentioned below:
(a) a protein consisting of the amino acid sequence of SEQ ID NO: 1 or 3:
(b) a protein consisting of an amino acid sequence having an amino acid sequence identity of 90% or higher to the amino acid sequence of SEQ ID NO: 1 or 3, and having an activity for catalyzing an epimerization reaction of a saccharide; and
(c) a protein consisting of an amino acid sequence derived from the amino acid sequence of SEQ ID NO: 1 or 3 by substitution, insertion, deletion and/or addition of one or several amino acids, and having an activity for catalyzing an epimerization reaction of a saccharide.

In this description, any of the proteins (a) to (c) mentioned above may be referred to as the protein contained in the enzyme preparation of the present invention or protein of the present invention.

"Epimerization of saccharide" or "epimerization for saccharide" is a reaction that inverts the optical configuration at one of the multiple chiral carbons of a saccharide, and specific examples thereof include, for example, epimerization (isomerization) of the hydroxyl group at the 2-position of a saccharide, and the reverse reaction thereof.

An enzyme that catalyzes epimerization is usually referred to as epimerase, and examples of well-studied epimerases include, for example, cellobiose 2-epimerase (CE). CE catalyzes a reaction of isomerizing the reducing terminal glucose residue of cellobiose into a mannose residue.

The amino acid sequence of SEQ ID NO: 1 is an amino acid sequence obtained on the basis of the genomic information of *Lunatimonas lonarensis* (NCBI Reference: WP_010853281.1). Although the amino acid sequence of SEQ ID NO: 1 has been annotated to be of the N-acylglucosamine 2-epimerase family, the specific properties thereof as an expressed protein have been unknown at the time of filing of the present application.

The inventors of the present invention obtained a protein consisting of the amino acid sequence of SEQ ID NO: 1 through expression thereof as a recombinant protein in a *Bacillus subtilis* expression system, and analyzed the enzymatic activity thereof. As a result, they found that the protein acts on at least glucose to produce mannose and galactose to produce talose. That is, the inventors of the present invention discovered that the protein consisting of the amino acid sequence of SEQ ID NO: 1 is an enzyme that catalyzes the epimerization of the hydroxyl group attached to the carbon at the 2-position of monosaccharides such as glucose, mannose, talose, and galactose. The amino acid sequence of SEQ ID NO: 3 is an amino acid sequence obtained from the amino acid sequence of SEQ ID NO: 1 by deleting a part deduced to be a signal peptide. The reaction of the epimerization of the hydroxyl group attached to the carbon at the 2-position of monosaccharides such as glucose, mannose, talose, and galactose to be catalyzed is reversible. For example, epimerization of the hydroxyl group attached to the carbon at the 2-position of glucose results in mannose, and epimerization of the hydroxyl group attached to the carbon at the 2-position of mannose results in glucose. Epimerization of the hydroxyl group attached to the carbon at the 2-position of talose results in galactose, and epimerization of the hydroxyl group attached to the carbon at the 2-position of galactose results in talose.

The amino acid sequence of SEQ ID NO: 1 derived from *Lunatimonas ronarensis* showed a primary amino acid sequence identity of about 50% to the amino acid sequences of known CEs. Specifically, the amino acid sequence of SEQ ID NO: 1 showed a primary amino acid sequence identity of 50% to CE derived from *Melioribacter roseus* (GenBank: AFN73749.1) (identity was determined by using ClustalW (Larkin M. A. et al., 2007, Bioinformatics, 2007; 23:2947-2948)).

The protein contained in the enzyme preparation of the present invention may also be a protein consisting of an amino acid sequence having an amino acid sequence identity of 90% or higher to the amino acid sequence of SEQ ID NO: 1 or 3, and having an activity for catalyzing an epimerization reaction of a saccharide. According to one embodiment, the protein contained in the enzyme preparation of the present invention may be a protein consisting of an amino acid sequence having an amino acid sequence identity of 91% or higher, 92% or higher, 93% or higher, 94% or higher, 95% or higher, 96% or higher, 97% or higher, 98% or higher, or 99% or higher to the amino acid sequence of SEQ ID NO: 1 or 3 and exhibiting an activity for catalyzing an epimerization reaction of a saccharide.

The amino acid sequence identity is defined as the percentage of amino acid residues that are identical between two aligned amino acid sequences to be compared, wherein gaps are introduced as required in order to obtain the maximum sequence identity. The amino acid sequence identity can be determined by using publicly available computer software such as, for example, BLAST, BLAST-2, ALIGN, and Megalign (DNASTAR) software.

Further, the protein contained in the enzyme preparation of the present invention may also be a protein consisting of an amino acid sequence derived from the amino acid sequence of SEQ ID NO: 1 or 3 by substitution, insertion, deletion and/or addition of one or several amino acids and having an activity for catalyzing an epimerization reaction of a saccharide. The range of "one to several" in the expression of "an amino acid sequence derived by substitution, insertion, deletion and/or addition of one or several amino acids" referred to in this description is not particularly limited, but means about, for example, 1 to 20, preferably 1 to 10, more preferably 1 to 7, even more preferably 1 to 5, or especially preferably 1 to 3.

For the method for producing the protein contained in the enzyme preparation of the present invention, see the section of <Production of protein> mentioned below.

The present invention further provides an enzyme preparation for catalyzing an epimerization reaction of a saccharide, which contains a culture supernatant of a transformed cell into which any of the polynucleotides (d) to (f) mentioned below has been introduced, and wherein the saccharide is any selected from the group consisting of glucose, mannose, talose and galactose:
(d) a polynucleotide consisting of the nucleotide sequence of SEQ ID NO: 2 or 4;
(e) a polynucleotide that hybridizes with a complementary strand of the polynucleotide consisting of the nucleotide sequence of SEQ ID NO: 2 or 4 under stringent conditions, and encodes a protein having an activity for catalyzing an epimerization reaction of a saccharide; and
(f) a polynucleotide having a nucleotide sequence identity of 90% or higher to the polynucleotide consisting of the nucleotide sequence of SEQ ID NO: 2 or 4, and encoding a protein having an activity for catalyzing an epimerization reaction of a saccharide.

The nucleotide sequence of SEQ ID NO: 2 is a nucleotide sequence encoding the amino acid sequence of SEQ ID NO: 1 obtained on the basis of the genomic information of *Lunatimonas lonarensis* (NCBI Reference: WP_010853281.1). The nucleotide sequence of SEQ ID NO: 4 is a sequence obtained from the nucleotide sequence of SEQ ID NO: 2 by deleting the nucleotide sequence encoding a part deduced to be the signal peptide.

A culture supernatant of a transformed cell that has been introduced with a polynucleotide that hybridizes with a complementary strand of the polynucleotide consisting of the nucleotide sequence of SEQ ID NO: 2 or 4 under stringent conditions, and encodes a protein having an activity for catalyzing an epimerization reaction of a saccharide also falls within the scope of the present invention. The term "polynucleotide that hybridizes with a complementary strand of the polynucleotide consisting of the nucleotide sequence of SEQ ID NO: 2 or 4 under stringent conditions" means a polynucleotide (e.g., DNA) that can be obtained by using a complementary strand of the polynucleotide consisting of the nucleotide sequence of SEQ ID NO: 2 or 4 as a probe in the colony hybridization method, plaque hybridization method, Southern blot hybridization method, or the like. The "stringent conditions" means, for example, such conditions that after Southern hybridization is performed, the filter is washed at 55 to 65°C with a 0.1 to 5 x SSC solution (composition of 1 x SSC: 150 mM sodium chloride, 15 mM sodium citrate) (Molecular Cloning: A Laboratory Manual 2^{nd} Ed. (Sambrook, Maniatis et al., Cold Spring Harbor Laboratory Press (1989)). According to one embodiment of the present invention, the stringent conditions consist of such conditions that after Southern hybridization is performed, the filter is washed at 60 to 65°C at salt concentrations corresponding to 0.1 to 1 x SSC, and 0.1% SDS.

A culture supernatant of a transformed cell introduced with a polynucleotide having a nucleotide sequence identity of 90% or higher, 91% or higher, 92% or higher, 93% or higher, 94% or higher, 95% or higher, 96% or higher, 97% or higher, 98% or higher, or 99% or higher to the polynucleotide consisting of the nucleotide sequence of SEQ ID NO: 2 or 4 and encoding a protein showing an activity for catalyzing an epimerization reaction of a saccharide also falls within the scope of the present invention. The sequence identity is defined as the percentage of nucleotides residues that are identical between two aligned nucleotide sequences to be compared, wherein gaps are introduced as required in order to obtain the maximum sequence identity. The nucleotide sequence identity can be determined by using publicly available computer software such as, for example, BLAST, BLAST-2, ALIGN, and Megalign (DNASTAR) software.

The transformed cell described above is preferably that of *Bacillus subtilis.* The culture supernatant of the present invention is substantially free from cells, and the cells can be removed with a filter or by centrifugation.

The culture supernatant of the present invention contains a protein that is an expression product of any of the polynucleotides (d) to (f) mentioned above. In this description, the protein that is an expression product of any of the polynucleotides (d) to (f) mentioned above and contained in the culture supernatant of the present invention may be referred to as the protein contained in the enzyme preparation of the present invention or the protein of the present invention.

The epimerization activity of the protein contained in the enzyme preparation of the present invention is evaluated by allowing the protein of the present invention to act on any selected from of glucose, mannose, talose and galactose as a substrate, and detecting the obtained epimerization reaction product.

The reaction product can be detected by using high performance liquid chromatography (HPLC) and an RI detector, charged particle detector, or the like. The detection can also be performed by using thin layer chromatography (TLC) and high performance ion exchange chromatography/pulsed amperometric detection (HPAEC-PAD).

Epimerization of glucose results in production of mannose, epimerization of mannose results in production of glucose, epimerization of talose results in production of galactose, and epimerization of galactose results in production of talose.

The enzyme preparation for catalyzing an epimerization reaction of a saccharide of the present invention can be used for catalyzing epimerization of hydroxyl group attached to the carbon at the 2-position of a reducing saccharide residue of mono- or disaccharides. In one embodiment of the present invention, the enzyme preparation for catalyzing an epimerization reaction of a saccharide of the present invention can be used to catalyze an epimerization reaction of any saccharide selected from at least, but not limited to, glucose, mannose, galactose and talose. This is because the protein contained in the enzyme preparation of the present invention acts on at least glucose, mannose and galactose. The protein contained in the enzyme preparation of the present invention can epimerize the hydroxyl group at the 2-position of monosaccharides.

The enzyme preparation for catalyzing an epimerization reaction of a saccharide of the present invention can be used for the production of a epimerization reaction product of a saccharide. Specifically, the enzyme preparation for catalyzing an epimerization reaction of a saccharide of the present invention can be used to produce mannose using glucose as a substrate, and can also be used to produce glucose using mannose as a substrate. In one embodiment of the present invention, the enzyme preparation of the present invention is preferably used to produce mannose using glucose as a substrate. This is because glucose is industrially mass-produced and inexpensive, and therefore glucose can be easily obtained for the production of mannose, which is a challenge for industrial production methods.

The enzyme preparation for catalyzing an epimerization reaction of a saccharide of the present invention can be used to produce talose using galactose as a substrate, and can also be used to produce galactose using talose as a substrate. In one embodiment of the present invention, the enzyme preparation of the present invention is preferably used to produce talose using galactose as a substrate. This is because galactose can be obtained by, for example, hydrolysis of lactose, which is abundant in nature, and therefore galactose can be easily procured for producing talose, which is one of the rare saccharides.

The enzyme preparation for catalyzing an epimerization reaction of a saccharide of the present invention may use or may not substantially use N-acylglucosamine as the substrate, but the preparation preferably does not substantially use N-acylglucosamine as the substrate. In this description, the expression that the preparation does not substantially use a specific saccharide as a substrate means that, for example, the relative activity for the specific saccharide as the substrate based on the activity for mannose as the substrate, which is taken as 100%, is 5% or lower, preferably 1% or lower, more preferably 0%, even more preferably that the activity for the above specific saccharide as the substrate is not measurable, and especially preferably, it means that the catalytic reaction with the above specific saccharide as the substrate does not occur completely.

The protein contained in the enzyme preparation of the present invention has a molecular weight in the range of 45 to 55 kDa or about 50 kDa as measured by SDS-PAGE. The molecular weight of the protein mentioned here is the molecular weight of the protein expressed with a plasmid inserted with a DNA encoding the protein by using *Bacillus subtilis* as the host cell. The theoretical value of the molecular weight of the proteins, of which molecular weights were measured to be in the range of 45 to 55 kDa and about 50 kDa in Examples 1 and 2, is 50.0 kDa.

The protein contained in the enzyme preparation of the present invention can act on at least, but are not limited to, glucose, mannose, talose and galactose to catalyze the epimerization reaction of these saccharides.

Some conventional cellobiose 2-epimerases produce mannose and fructose from glucose (Patent documents 3 and 4). However, the protein contained in the enzyme preparation of the present invention can convert glucose mainly to mannose. In the reaction system using the protein of the present invention, a small amount of fructose may be produced from glucose, but only in trace amounts.

The protein contained in the enzyme preparation of the present invention can produce mannose from glucose more efficiently compared with conventional cellobiose 2-epimerases. Specifically, when the protein contained in the enzyme preparation of the present invention is allowed to act on the substrate glucose under the same conditions as described in the examples, it can produce mannose at a content of 1.3 times or higher, or a content of 1.4 times or higher, 1.5 times or higher, 1.6 times or higher, 1.7 times or higher, 1.8 times or higher, 1.9 times or higher, or 2.0 times or higher, in the reaction product compared with CE derived from *Melioribacter roseus* described in Patent document 6.

The enzyme preparation of the present invention may be an enzyme composition containing the aforementioned protein having an activity for catalyzing an epimerization reaction of a saccharide in a state of an isolated and purified protein as an active ingredient, and may contain further ingredients in addition to the aforementioned protein having an activity for catalyzing an epimerization reaction, so long as such ingredients are those not inhibiting the epimerization reaction of a saccharide. These ingredients may be ingredients normally used in enzyme preparations, for example, a buffer, stabilizer, excipient, and so forth. Such further ingredients are known from the prior art and are well known to those skilled in the art. Form of the enzyme preparation of the present invention is not also particularly limited, and it may be in the form of solid (e.g., powder form) or liquid. The enzyme preparation of the present invention can be used by, for example, adding it in the form of solid or liquid to a solution of a saccharide substrate.

The enzyme preparation of the present invention can be provided as, for example, an enzyme preparation in which the protein having an activity for catalyzing an epimerization reaction of a saccharide of the present invention is immobilized on an immobilization carrier. By using such an immobilized enzyme preparation, for example, the production of a reaction product can be carried out at a high substrate concentration and high temperature, and the production of a reaction product can also be carried out in a scheme using a bioreactor.

The immobilization carrier mentioned above is not particularly limited, and, for example, a carrier on which the protein having an activity for catalyzing an epimerization reaction of a saccharide can be adsorbed or cross-linked, and the activity of the protein of the present invention is retained can be used.

Examples of the immobilization carrier mentioned above include, for example, anion-exchange carriers, cation-exchange carriers, hydrophobic carriers, and so forth. Specific examples of the immobilization carrier mentioned above include, for example, ion-exchange gels. Examples of the ion-exchange gels include those of the DIAION (registered trademark) series such as DIAION (registered trademark) SK1B, DIAION (registered trademark) PK212, DIAION (registered trademark) HPA25, and DIAION (registered trademark) UBK555 (manufactured by Mitsubishi Chemical Corporation); those of the SEPABEADS (registered trademark) series such as SEPABEADS (registered trademark) SP SP-207, and SEPABEADS (registered trademark) SP-850 (manufactured by Mitsubishi Chemical); those of the DUOLITE series such as DUOLITE A568, DUOLITE PWA7, and DUOLITE XAD761 (manufactured by Sumika Chemtex Co. Ltd.), and so forth.

The enzyme preparation of the present invention can also be used for a saccharide other than glucose, mannose, talose, and galactose, so long as the protein contained in the enzyme preparation of the present invention can catalyze the epimerization of such a saccharide.

Form of the immobilization carrier is not particularly limited, and it may be in the form of, for example, membrane, beads, plate, or the like.

The method for immobilizing the protein having an activity for catalyzing an epimerization reaction of a saccharide onto the immobilization carrier is not particularly limited. For example, the immobilization can be attained by adding the protein and the immobilization carrier to a solvent such as a buffer, and shaking the resulting mixture.

### (Method for producing epimerization reaction product of saccharide)

The present invention provides a method for producing an epimerization reaction product of a saccharide, which comprises the step of allowing the enzyme preparation for catalyzing an epimerization reaction of a saccharide of the present invention to act on a saccharide substrate to obtain an epimerization reaction product of the saccharide.

The enzyme preparation of the present invention can be allowed to act on a saccharide substrate by, for example, preparing an aqueous solution of the saccharide substrate, adjusting pH thereof as required, and then adding the enzyme preparation of the present invention to the aqueous solution. The enzyme preparation of the present invention can also be allowed to act on a saccharide substrate by immobilizing the protein having an activity for catalyzing an epimerization reaction of a saccharide of the present invention on a carrier to form the enzyme preparation, and then contacting an aqueous solution of a saccharide substrate with the enzyme preparation. This is because the epimerization reaction of a saccharide using the enzyme preparation of the present invention can be carried out in a reaction solution.

The saccharide substrate that can be used in the method for producing an epimerization reaction product of a saccharide may be any selected from glucose, mannose, galactose, and talose, but is not limited to these, and any other saccharide can be used as the saccharide substrate so long as the protein contained in the enzyme preparation of the present invention can catalyze epimerization thereof. As the saccharide substrate, a pure product may be used, but a saccharide composition containing other saccharides may also be used for the reasons of cost etc. Specifically, for example, when glucose is used as the main substrate, a saccharide composition containing maltose or maltotriose, which are produced by the decomposition of starch, in addition to glucose may also be used.

The reaction temperature at the time of allowing the protein contained in the enzyme preparation of the present invention to act on a saccharide substrate is not particularly limited, so long as the reaction temperature is in such a temperature range that the enzymatic activity can be exhibited. The protein contained in the enzyme preparation of the present invention can catalyze the reaction at temperatures of 53°C or lower when the reaction is allowed at pH 6.0, as described in the examples. Some conventional epimerases have limitations regarding their use on an industrial scale due to their thermal stability. However, the protein contained in the enzyme preparation of the present invention exhibits high temperature resistance, and shows thermal stability suitable for use on an industrial scale.

Therefore, the reaction temperature (liquid temperature of the reaction solution) at the time of allowing the enzyme preparation of the present invention to act can be set within a wide temperature range, such as 35 to 55°C. In industrial scale reaction systems, precise temperature control may be difficult, but the protein of the present invention is advantageous in that it can be stably used over a wide temperature range.

The reaction temperature can be set in the range of 35 to 55°C, but it is preferably set it in the range of 45 to 53°C. This is because, if the reaction temperature is 45°C or higher, contaminant bacteria that utilize saccharides as a nutrient cannot easily grow, and the protein of the present invention shows temperature stability at 53°C or lower.

In the method for producing an epimerization reaction product of a saccharide, the enzyme preparation of the present invention can be used in combination with another enzyme, as described later. When another enzyme is used in combination, the reaction temperature may be within a temperature range in which the proteins contained in the enzyme preparation of the present invention act stably. Since the protein contained in the enzyme preparation of the present invention is stable over a wide temperature range as described above, the temperature can often be set in such a range that the activities of all enzymes used in the reaction system can fully be utilized, taking into consideration the temperatures at which the other enzymes used in combination can be stably activated.

The reaction temperature may not be constant throughout the whole reaction period, and can be appropriately adjusted. For example, if it is desirable to increase the activity of another enzyme used in combination in an early stage of the reaction period, the temperature in the early stage of the reaction can be set to be in a temperature range in which the activity of that enzyme is increased, and in the middle or late stage of the reaction period, the temperature can be set to be in a temperature range in which the activity of the protein contained in the enzyme preparation of the present invention is increased.

The pH of the reaction solution in which the protein contained in the enzyme preparation of the present invention is allowed to act on a saccharide substrate is not particularly limited so long as it is in such a pH range that the enzyme activity can be exhibited, but it can be set in the range of 5.5 to 8.5. This is because the protein contained in the enzyme preparation of the present invention can catalyze the reaction at least in the pH range of 6.0 to 8.0, as described in the examples. In order to efficiently obtain a reaction product of an epimerization of a saccharide, the pH of the reaction solution at the time of allowing the protein contained in the enzyme preparation of the present invention to act is preferably set in the range of 6.0 to 8.0. The pH of the reaction solution can be adjusted as required by addition of an acid or alkali.

The reaction time of the reaction of the substrate and the protein contained in the enzyme preparation of the present invention in the method for producing an epimerization reaction product of a saccharide can be appropriately determined in consideration of the reaction temperature, concentration of the substrate, characteristics of another enzyme to be used when such another enzyme is used in combination, and so forth. The optimal reaction time for efficiently producing an epimerization reaction product can also be determined on the basis of conventional techniques in this field. Specific examples of the reaction time include, but are not intended to be limited to, 10 minutes to 72 hours. During the reaction, the enzyme preparation of the present invention can be added as required.

The substrate concentration in the reaction solution is not particularly limited. For example, a higher substrate concentration is more economically advantageous, so long as it is within the range in which the above enzymatic reaction can proceed. When glucose is used as the substrate, the glucose concentration is in the range of 0.1 to 300 g per 100 g of solvent. The solvent of the above reaction solution is not particularly limited, and examples include, for example, water, buffer, and so forth. If the solubility of the selected substrate in the solvent is low, for example, it may not be completely dissolved at the desired concentration. When an enzyme reaction is performed batch-wise, for example, the substrate need not be completely dissolved at an early stage of the reaction, and the concentration of the substrate can be chosen to be in such a range that the substrate is dissolved at the end of the enzymatic reaction. However, when a column packed with an immobilized enzyme preparation is used for the enzyme reaction, for example, it is preferred that the substrate should be completely dissolved to avoid pressure loss due to clogging of the column. In addition, when a column packed with an immobilized enzyme preparation is used for the enzyme reaction, for example, it is preferred that the substrate solution should be continuously passed through the column by circulating the substrate solution. When mannose is produced from glucose as the substrate by using the enzyme preparation of the present invention, starch syrup, powdered syrup, hydrol (centrifugation supernatant produced during the production of crystalline glucose), and so forth can be used. Further, mannose can also be produced from glucose by using a carbohydrate containing glucose as a constituent saccharide such as dextrin and starch as a starting material, while glucose as a substrate is produced from the starting material by adding a hydrolytic enzyme together with the enzyme preparation of the present invention.

As a specific example of the production of the epimerization reaction product using the enzyme preparation of the present invention, when glucose is used as the substrate, mannose can be produced by preparing an aqueous glucose solution having a solid concentration of about 1 to 65%, adjusting pH of the solution to about 6.0 to 8.0 using an acid or alkali as required, adding the enzyme preparation of the present invention to the aqueous solution, and maintaining the resulting mixture at a temperature in the range of 30 to 53°C for about 1 to 72 hours.

In the method for producing an epimerization reaction product of a saccharide, the enzyme preparation of the present invention may be used in combination with another enzyme. The other enzyme may be, but not limited to, an enzyme that produces a saccharide that can be a substrate for the enzyme having an activity for catalyzing an epimerization reaction of a saccharide of the present invention. For example, an enzyme that hydrolyzes carbohydrates containing galactose as a constituent saccharide, such as lactose, can be used as the other enzyme. By using the enzyme preparation of the present invention (i.e., enzyme preparation for catalyzing an epimerization reaction of a saccharide) together with the hydrolytic enzyme as the other enzyme, galactose can be generated from carbohydrates containing galactose as a constituent saccharide, such as lactose, by hydrolysis, and talose can be generated from galactose by epimerization at the same time.

An aqueous saccharide solution containing an epimerization reaction product is obtained by the production method mentioned above. The aqueous saccharide solution containing an epimerization reaction product can be decolorized, desalted, purified, etc. as required by using conventional methods. The aqueous saccharide solution containing an epimerization reaction product can be subjected to resin fractionation, precipitation with an organic solvent such as ethanol, chromatographic fractionation, ultrafiltration membrane treatment or the like to remove unreacted substrate and thereby increase the purity of the epimerization reaction product. The epimerization reaction product can be purified more efficiently by a single kind of purification treatment operation or a combination of several kinds of such operations.

As another aspect, the present invention provides a method for producing an epimerization reaction product of a saccharide, comprising the step of allowing the enzyme preparation for catalyzing an epimerization reaction of a saccharide of the present invention to act on a saccharide substrate under a condition of pH 5.5 to 7.0 to obtain an epimerization reaction product of the saccharide, wherein byproducts in the reaction product are suppressed to not more than 5% of the objective epimerization reaction product. For example, mannose can be produced by allowing the enzyme preparation for catalyzing an epimerization reaction of a saccharide of the present invention to act on glucose under a condition of pH 5.5 to 7.0, and in this method, fructose as a byproduct in the reaction product can be suppressed to 5% or less of mannose. pH as the above pH condition is preferably in the range of 5.5 to 6.5. The byproducts in the reaction product is preferably suppressed to 4.5% or less, 4.0% or less, 3.5% or less, 3.0% or less, 2.5% or less, 2.0% or less, 1.5% or less, 1.0% or less, or 0.5% or less of the objective epimerization reaction product.

The present invention provides a method for producing a food, feed, feed for fish culture, cosmetic, or medicament, which comprises the step of producing the food, feed, feed for fish culture, cosmetic, or medicament by using an epimerization reaction product of a saccharide obtained by implementing the method for producing an epimerization reaction product of a saccharide.

The epimerization reaction product of a saccharide can be obtained by implementing the method for producing an epimerization reaction product of a saccharide described above.

In the step of producing a food, feed, feed for fish culture, cosmetic, or medicament by using an epimerization reaction product of a saccharide obtained by implementing the method for producing an epimerization reaction product of a saccharide, the food, feed, feed for fish culture, cosmetic, or medicament can be produced by using the epimerization reaction product of a saccharide as one of the raw materials, or the epimerization reaction product of a saccharide itself can be prepared in an appropriate form (powder, liquid, etc.), and provided as a food, feed, feed for fish culture, cosmetic, or medicament.

Examples of the food produced by the method of the present invention include, but are not limited to, various carbohydrate foods (bread, noodles, rice, rice cakes), various Japanese confectioneries (senbei, arare, okoshi, gyuhi, mochi, manju, dorayaki, uiro, bean pastes, yokan, mizu-yokan, nishikidama, Castella sponge cake, amedama candy, etc.), various Western confectioneries (bread, biscuit, cracker, cookie, pie, doughnut, steamed cake, pudding, jelly, mousse, bavarois, custard cream, cream puff, waffle, sponge cake, chocolate, chewing gum, caramel, nougat, candy, syrups, etc.), various ice confectioneries (ice cream, sherbet, gelato, shaved ice, etc.), various pastelike foods (flour paste, peanut paste, margarine, fruit paste, etc.), various beverages (beverages containing fruit juice, fruit juice, vegetable juice, cider, ginger ale, isotonic beverages, amino acid beverages, jelly beverages, coffee beverages, green tea, black tea, oolong tea, barley tea, dairy beverages, lactobacillus beverages, cocoa, beer, low-malt beer, malt-free beer-like alcoholic beverage, non-alcoholic beverages, beer-flavored beverages, liqueur, chuhai, sake, fruit wine, distilled spirits, nutritional drinks, health drinks, powdered beverages, etc.), processed fruit and vegetable foods (jam, marmalade, fruits and vegetables soaked in syrup, sugar confections, pickles, etc.), various dairy products (cheese, yogurt, butter, condensed milk, powdered milk, etc.), powdered food (powdered soup, powdered mousse, powdered jelly, powdered sweetener, etc.), nutritional foods, diet foods, nutritional foods for sports, liquid foods, semi-solid liquid foods, nursing care foods, swallowing foods, and so forth.

Examples of the feed and feed for fish culture produced by the method of the present invention include, but are not limited to, feeds and feeds for fish culture for livestock, poultry, fish and shellfish, and insects (honeybees, silkworms, etc.). Examples of the form thereof include, but are not limited to, powder, pellet, tablet, kneaded feed, capsule, and so forth.

Examples of the cosmetic produced by the method of the present invention include, but are not limited to, moisturizer, beautifying agent, and so forth. Examples of the form thereof include milky liquid, cream, emulsion, and so forth.

Examples of the medicament produced by the method for producing a medicament of the present invention include, but are not limited to, anti-obesity agent, blood glucose level increase inhibitor, and so forth, and examples of the form thereof include tablet, powder, liquid, capsule, and so forth.

### <Production of protein>

The method for obtaining the protein contained in the enzyme preparation of this invention is not particularly limited, and the protein may be a protein synthesized by chemical synthesis or a recombinant protein produced by a genetic recombination technique. The production of a recombinant protein will be explained below.

A protein having the amino acid sequence of SEQ ID NO: 1 or 3, an amino acid sequence having an amino acid sequence identity of 90% or higher to the amino acid sequence of SEQ ID NO: 1 or 3, or an amino acid sequence derived from the amino acid sequence of SEQ ID NO: 1 or 3 by substitution, insertion, deletion and/or addition of one or several amino acids can be prepared by genetic engineering techniques. For example, the protein can be produced by transforming a host cell with a gene encoding the amino acid sequence of SEQ ID NO: 1 or 3 as a DNA molecule that can replicate in the host cell, or a DNA molecule that can be incorporated into a chromosome and contains the gene in an expressible state, especially in a form thereof inserted into an expression vector, and culturing the host cell. Such a DNA molecule can be obtained by incorporating a DNA fragment encoding the amino acid sequence of SEQ ID NO: 1 or 3, an amino acid sequence having an amino acid sequence identity of 90% or higher to the amino acid sequence of SEQ ID NO: 1 or 3, or an amino acid sequence derived from the amino acid sequence of SEQ ID NO: 1 or 3 by substitution, insertion, deletion and/or addition of one or several amino acids into a vector molecule. According to a preferred embodiment of the present invention, this vector is a plasmid. The DNA molecules used in the present invention can be prepared according to the methods described in Molecular Cloning: A Laboratory Manual 2nd Ed. (Sambrook, Maniatis, et al., Cold Spring Harbor Laboratory Press (1989)).

The vector that can be used in the present invention can be appropriately selected from viruses, plasmids, cosmid vectors, and so forth, taking into consideration the type of host cell to be used. For example, examples include plasmids of the pJEXOPT2 series (see Japanese Patent No. 5126879), and plasmids of the pHT series for *Bacillus subtilis* as the host cell; bacteriophages of the λ phage type and plasmids of the pET series, pUC series, pCold series, and pGEX series for *E. coli* as the host cell; vectors of the YEp, YCp, and YIP series, and pLeu4, pPPLeu4, pJPLeu series (described in Japanese Patent Unexamined Publication (Kokai) No. No. Hei 4-218382) for yeast as the host cell, and so forth, but are not limited to these. The plasmid may contain a marker for selecting transformants, and the selection marker may be a drug resistance marker or nutrient requirement marker gene, but is not limited to these.

Furthermore, the expression vector that can be used in the present invention can have DNA sequences necessary for expression of the enzyme gene, for example, promoter, terminator, ribosome binding site, transcriptional regulatory signal such as transcription termination signal, and translational regulatory signal. As the promoter, promoters of subtilisin gene, SPAC gene, and so forth can be used in *Bacillus subtilis,* and promoters of alcohol dehydrogenase gene (ADH), acid phosphatase gene (PHO), galactose metabolic genes (GAL), glyceraldehyde triphosphate dehydrogenase gene (GAP), and so forth can be used in yeast, but it is not limited to these. It is preferable to use a signal peptide, because it has an advantage that it makes the objective enzyme to be secreted in culture supernatant, and therefore makes the purification thereof easier. The signal peptide can be replaced with one derived from *Bacillus subtilis* or yeast (e.g., invertase signal, acid phosphatase signal, λ-factor signal, etc.). In addition, in *E. coli,* more efficient expression can be devised by expressing molecular chaperone at the same time by using the cspA promoter or the like in addition to the commonly used lac promoter and T7 promoter.

For the culture of the transformed host cells, common methods for the used host cells can be used. The enzyme is usually generated and accumulated in the cells or extracellular culture medium after about 1 to 4 days of culture. As for the culture conditions (medium, pH, temperature, etc.), for example, the culture temperature is generally 25 to 37°C for bacteria, 25 to 30°C for yeast, and about 37°C for eukaryotic cells. For the culture conditions, Manual of Gene Expression Experiments (Kodansha) and so forth can be referred to.

As the host cells, bacteria such as coli bacilli and hay bacilli, and yeast such as *Candida utilis, Saccharomyces cerevisiae* and *Pichia pastoris,* as well as *Rhizopus niveus, Rhizopus delemar,* and higher eukaryotes (such as CHO cells) can be used. As the hay bacilli, it is preferable to use a microorganism belonging to the genus *Bacillus.* It is known that *Bacillus* bacteria include strains that secrete proteins outside the cells of the bacteria (e.g., *Bacillus subtilis,* etc.). Strains that secrete almost no protease are also known, and it is also preferable to use such strains as the host. In the present invention, yeast, filamentous fungi or bacteria are preferred as the host cells, but bacteria are more preferred, and *Escherichia coli* and *Bacillus subtilis* are especially preferred.

The protein produced by the transformant can be isolated and purified by an appropriate combination of known separation and purification methods. These separation and purification methods include, for example, methods utilizing differences in solubility such as salt precipitation and solvent precipitation, methods utilizing differences in molecular weights such as dialysis, ultrafiltration, gel filtration and SDS-polyacrylamide electrophoresis, methods utilizing differences in electric charges such as ion exchange chromatography, methods utilizing differences in hydrophobicity such as hydrophobic chromatography and reverse phase chromatography, and methods utilizing differences in isoelectric point such as isoelectric focusing, as well as affinity chromatography, and so forth. For the purification methods described in the examples and other general separation and purification methods, for example, Basic Experimental Methods for Proteins and Enzymes (Nankodo) can be referred to.

### Examples

The present invention will be more specifically explained with reference to the following examples. However, the present invention is not limited to these examples. In this description, unless especially stated, "%" is mass-based, and numerical ranges are mentioned so as to include their end points. Further, unless especially stated, the operating procedures were performed according to the methods described in Molecular Cloning: A Laboratory Manual 2nd Ed. (Sambrook, Maniatis et al., Cold Spring Harbor Laboratory Press (1989)).

In the examples, a gene encoding a protein of unidentified function, presumed to be N-acylglucosamine 2-epimerase (AGE) (NCBI Reference: WP_010853281.1) was cloned from *Lunatimonas lonarensis,* a recombinant protein was obtained with it, and the recombinant protein was characterized for the enzymatic activity thereof.

### Example 1: Extracellular expression of the enzyme of the invention using Bacillus subtilis as host (1)

### 1-1. Construction of expression plasmid

Various microorganisms were searched for novel enzymes, and the amino acid sequence derived from *Lunatimonas lonarensis* (NCBI Reference: WP_010853281.1) was selected, and functional analysis thereof was conducted. The amino acid sequence thereof (SEQ ID NO: 1) and the nucleotide sequence (SEQ ID NO: 2) of the gene encoding the amino acid sequence (hereafter referred to as the objective gene 1) are shown in Fig. 1A and Fig. 1B, respectively. The amino acid sequence of SEQ ID NO: 3 shown in Fig. 1C corresponds to the amino acid sequence of SEQ ID NO: 1 of which part presumed to be a signal peptide is deleted. The nucleotide sequences of SEQ ID NOS: 2 and 4 had been codon-corrected to optimize them for favorable expression in *Bacillus subtilis.*

An expression plasmid for expressing the objective gene 1 in *Bacillus subtilis* was constructed. First, the objective gene was amplified by PCR using a plasmid consisting of the pUC57 vector inserted with the nucleotide sequence of SEQ ID NO: 2 as a template, a primer having a nucleotide sequence homologous to the end of the signal peptide sequence of the vector for sense strand amplification, and a primer containing a part of the terminator sequence for anti-sense strand amplification. The composition of the reaction solution for the PCR amplification is shown below.

| | |
|---|---|
| 2 × Primestar Max Premix (Takara Bio) | 50 µL |
| 10 µM Primer (LlAGE-Fw) | 2 µL |
| 10 µM Primer (LlAGE-Rv) | 2 µL |
| 1 ng/µL Template | 2 µL |
| H₂O | 44 µL |

The primers used are shown in Table 1.

**[Table 1]**

| Primer | Sequence (5'→3') |
|---|---|
| LlAGE-Fw (sense) | ACTGCTCTTGGATCCTCTTGCAGAAATTCAG (SEQ ID NO: 5) |
| LlAGE-Rv (anti-sense) | GATCTGCAGGTCGACTTAATTTTCGCGCAG (SEQ ID NO: 6) |

The PCR amplification reaction program was as follows: initial incubation at 96°C for 1 minute, followed by 35 cycles of 98°C for 10 seconds, 55°C for 5 seconds, and 72°C for 10 seconds, and further incubation at 72°C for 5 minutes. The obtained PCR product was subjected to agarose gel electrophoresis, the band corresponding to the amplified fragment (1,299 bp) was cut out from the gel, and the fragment was extracted and purified by using illustra^{™} GFX^{™} PCR DNA and Gel Band Purification Kit (GE Healthcare).

To prepare a linearized plasmid for use in the In-Fusion (registered trademark) cloning reaction, PCR was performed by using a plasmid obtained by modifying the vector pJEXOPT2 (see Japanese Patent No. 5126879) for optimization for this example as the template, and the primers shown in Table 2. For anti-sense strand amplification, a primer designed for amplification from the end of the signal peptide sequence of the vector was used.

The primers used are shown in Table 2.

**[Table 2]**

| Primer | Sequence (5'→3') |
|---|---|
| pJEXOPT2-Fw (sense) | GTCGACCTGCAGATCTCTAG (SEQ ID NO: 7) |
| pJEXOPT2-Rv (anti-sense) | GGATCCAAGAGCAGTGG (SEQ ID NO: 8) |

The composition of the PCR amplification reaction solution was the same as that used for the amplification of the objective gene 1, except for the primers and template. The PCR amplification reaction program was as follows: initial incubation at 96°C for 1 minute, followed by 35 cycles of 98°C for 10 seconds, 55°C for 5 seconds, and 72°C for 35 seconds, and further incubation at 72°C for 5 minutes. The obtained PCR product was subjected to agarose gel electrophoresis, the band corresponding to the amplified fragment (6,935 bp) was cut out from the gel, and the fragment was extracted and purified by using illustra^{™} GFX^{™} PCR DNA and Gel Band Purification Kit (GE Healthcare). The amplified DNA fragment of the objective gene 1 and the amplified fragment of the vector pJEXOPT2 were ligated by using In-Fusion HD Cloning Kit (Takara Bio). The ligation reaction was carried out by holding them at 50°C for 15 minutes.

*E. coli* DH5α was transformed by using 2.5 µL of the ligation reaction solution, and the plasmid DNA was prepared from the culture medium in which the *E. coli was* cultured by using illustra^{™} plasmidPrep Mini Spin Kit (GE Healthcare). The obtained plasmid was designated as "plasmid for expression of the enzyme of the present invention".

### 1-2. Expression of recombinant protein

The plasmid for expression of the enzyme of the present invention described above was introduced into *Bacillus subtilis* ISW1214 made into protoplasts (Takara Bio), and culture was performed at 30°C for 2 days in the DM3 regeneration agar medium (composition: 8.1% sodium succinate, 1% agar, 0.5% casamino acid, 0.5% yeast extract, 0.15% potassium dihydrogen phosphate, 0.35% dipotassium hydrogen phosphate, 0.5% glucose, 0.4% magnesium chloride, 0.01% bovine serum albumin, 0.001% methionine, and 0.001% leucine) containing 7.5 µg/mL tetracycline. By using the obtained colonies, the bacterium was pre-cultured, and then further cultured for 72 hours as the main culture (cultured as described in Japanese Patent No. 5126879, provided that the composition of the medium was modified). The culture medium was centrifuged (5,000 x g, 4°C, 5 minutes), the supernatant was filtered through a filter having a pore size of 0.45 µm (Merck), and the filtrate was used as the solution 1 of the enzyme of the present invention. This solution 1 of the enzyme of the present invention was subjected to SDS-PAGE, and a band around the desired size (50,000) was confirmed (Fig. 3).

### Example 2: Extracellular expression of the enzyme of the invention using Bacillus subtilis as host (2)

The experiment of Example 2 was performed by using the plasmid for expression of the enzyme of the present invention constructed in Example 1, 1-1.

### 2-1. Expression of recombinant protein

Introduction of the plasmid for expression of the enzyme of the present invention into *Bacillus subtilis* ISW1214 (Takara Bio) and culture were performed in the same manners as those of Example 1, 1-2. The obtained culture medium was centrifuged (5,000 x g, 4°C, 5 minutes), the supernatant was filtered through a filter having a pore size of 0.45 µm (Merck), and the filtrate was used as culture supernatant. This culture supernatant was subjected to SDS-PAGE, and a band around the desired size (50,000) was confirmed (Fig. 4). This culture supernatant was further concentrated about 10-fold by using Amicon Ultra-15 Centrifugation Filter Unit (Merck) to obtain the solution 2 of the enzyme of the present invention.

### Comparative example 1: Preparation of enzyme solution for comparison

### 1. Construction of expression plasmid

For functional comparison of the epimerase derived from *Lunatimonas lonarensis* described in Example 1, CE derived from *Melioribacter roseus* (NCBI Reference of amino acid sequence: AFN73749.1) described in Patent document 6 was extracellularly expressed in *Bacillus subtilis.* This amino acid sequence (SEQ ID NO: 9) and the nucleotide sequence of the gene encoding the amino acid sequence (hereafter referred to as comparative gene, SEQ ID NO: 10) are shown in Fig. 2A and Fig. 2B, respectively. The nucleotide sequence of SEQ ID NO: 10 had been codon-corrected to optimize it for favorable expression thereof in *Bacillus subtilis.*

An expression plasmid for expressing the comparative gene in *Bacillus subtilis* was constructed. First, the comparative gene was amplified by PCR using a plasmid consisting of the pUC57 vector inserted with the nucleotide sequence of SEQ ID NO: 10 as a template, a primer containing a nucleotide sequence homologous to the end of the signal peptide sequence of the vector for sense strand amplification, and a primer containing a part of the terminator sequence for anti-sense strand amplification. The composition of the reaction solution for the PCR amplification is shown below.

| | |
|---|---|
| 2 × Primestar Max Premix (Takara Bio) | 50 µL |
| 10 µM Primer (MROSCE-Fw) | 2 µL |
| 10 µM Primer (MROSCE-Rv) | 2 µL |
| 1 ng/µL Template | 2 µL |
| H₂O | 44 µL |

The primers used are shown in Table 3.

**[Table 3]**

| Primer | Sequence (5'→3') |
|---|---|
| MROSCE-Fw (sense) | ACTGCTCTTGGATCCATGGGCATTCTG (SEQ ID NO: 11) |
| MROSCE-Rv (anti-sense) | GATCTGCAGGTCGACTTATTTTTCAACAGCCTG (SEQ ID NO: 12) |

The PCR amplification reaction program was as follows: initial incubation at 96°C for 1 minute, followed by 35 cycles of 98°C for 10 seconds, 55°C for 5 seconds, and 72°C for 10 seconds, and further incubation at 72°C for 5 minutes. The obtained PCR product was subjected to agarose gel electrophoresis, the band corresponding to the amplified fragment (1,383 bp) was cut out from the gel, and the fragment was extracted and purified by using illustra^{™} GFX^{™} PCR DNA and Gel Band Purification Kit (GE Healthcare).

A linearized plasmid for use in the In-Fusion (registered trademark) cloning reaction was prepared by an operation similar to that of Example 1, 1-1, and the amplified DNA fragment of the comparative gene and the amplified fragment of a plasmid obtained by modifying the vector pJEXOPT2 (see Japanese Patent No. 5126879) for optimization for this example were ligated by using In-Fusion HD Cloning Kit (Takara Bio). The ligation reaction was carried out by holding them at 50°C for 15 minutes.

*E. coli* DH5α was transformed by using 2.5 µL of the ligation reaction solution, and a plasmid DNA was prepared from the culture medium in which the *E. coli* was cultured by using illustra^{™} plasmidPrep Mini Spin Kit (GE Healthcare). The obtained plasmid was designated as "plasmid for expression of the comparative enzyme".

### 2. Expression of recombinant protein

The plasmid for expression of the comparative enzyme described above was introduced into *Bacillus subtilis* ISW1214 made into protoplasts (Takara Bio), and culture was performed at 30°C for 2 days in a regeneration agar medium (composition: 8.1% sodium succinate, 1% agar, 0.5% casamino acid, 0.5% yeast extract, 0.15% potassium dihydrogen phosphate, 0.35% dipotassium hydrogen phosphate, 0.5% glucose, 0.4% magnesium chloride, 0.01% bovine serum albumin, 0.001% methionine, and 0.001% leucine) containing 7.5 µg/mL tetracycline. By using the obtained colonies, pre-culture and main culture were performed in the same manners as those of Example 1, 1-2. The obtained culture medium was centrifuged (5,000 x g, 4°C, 5 minutes), the supernatant was filtered through a filter having a pore size of 0.45 µm (Merck), and the filtrate was used as the solution of the comparative enzyme.

### Comparative Example 2: Preparation of empty vector control (negative control)

As a negative control, culture supernatant of the cells transformed with an empty vector was prepared. A plasmid obtained by modifying of the vector pJEXOPT2 (see Japanese Patent No. 5126879) for optimization for this example was introduced into *Bacillus subtilis* ISW1214 made into protoplasts (Takara Bio), and culture was performed at 30°C for 2 days in a regeneration agar medium (composition: 8.1% sodium succinate, 1% agar, 0.5% casamino acid, 0.5% yeast extract, 0.15% potassium dihydrogen phosphate, 0.35% dipotassium hydrogen phosphate, 0.5% glucose, 0.4% magnesium chloride, 0.01% bovine serum albumin, 0.001% methionine, and 0.001% leucine) containing 7.5 µg/mL tetracycline. By using the obtained colonies, pre-culture and main culture were performed in the same manners as those of Example 1, 1-2. The obtained culture medium was centrifuged (5,000 x g, 4°C, 5 minutes), the supernatant was filtered through a filter having a pore size of 0.45 µm (Merck), and the filtrate was used as the empty vector control (negative control).

### Example 3: TLC analysis of enzyme reaction products

In this example, substrates on which the enzyme of the present invention can act were verified. Mannose and galactose were used as the substrate for the enzyme reaction.

A reaction solution in a volume of 100 µL consisting of 10 µL of 50 mM HEPES-NaOH buffer (pH 7.0), 10 µL of a 500 mM solution of the substrate, 10 µL of the solution 1 of the enzyme of the present invention, and 70 µL of ultrapure water was maintained at 37°C for 24 hours, of which 0.5 µL was then subjected to thin layer chromatography (TLC).

The developing solvent was a mixture of 2-propanol/1-butanol/water (mixing ratio was 2:2:1 in the order (all by volume)).

Saccharides were detected by spraying a mixture of anisaldehyde/sulfuric acid/acetic acid (mixing ratio was 1:2:97 in the order (all by volume)) as a coloring agent onto the TLC plate and then heating it.

The results are shown in Fig. 5. For both the substrates, differences were observed in the migration distances of the saccharide (substrate) observed before the reaction and the saccharide observed after the reaction. In the analysis using mannose as the substrate, a spot consistent with the mobility of glucose was observed in the sample after the reaction. In the analysis using galactose as the substrate, a spot consistent with the mobility of talose was observed in the sample after the reaction. It was found that the enzyme of the present invention derived from *Lunatimonas lonarensis* act on at least mannose and galactose.

### Example 4: Mannose synthesis test

### 4-1. Method

A mannose synthesis test using glucose as a substrate was performed with the solution 1 of the enzyme of the present invention and the comparative enzyme solution. That is, 1 mL of a reaction solution containing 10.3 to 330.0 µL/g-ds of the enzyme (the solution 1 of the enzyme of the present invention or the comparative enzyme solution), 50% glucose, 40 mM buffer (MES-NaOH buffer (pH 6.0), sodium phosphate buffer (pH 7.0) or HEPES-NaOH buffer (pH 8.0)), and 0.02% sodium azide was kept at 50°C for 24 to 72 hours. In order to investigate isomerization caused by coexisting buffer components, a reaction solution using the empty vector control was also prepared and maintained in the same manner as that described above. The reaction solutions collected after various reaction times were adjusted to pH 4.0 or lower, and boiled for 10 minutes to terminate the reaction. Then, 10 µL of each reaction solution was diluted by mixing it with 800 µL of pure water.

The saccharide composition of the product obtained with the enzyme of the present invention using glucose as the substrate was analyzed by HPLC. The analysis conditions were as follows: column, HILICpak VG-50 4E (Shodex), and HILICpak VG-50G 4A (Shodex); eluent, 80% acetonitrile; flow rate, 0.6 mL/min; column temperature, 40°C; and detector, charged particle detector.

### 4-2. Results

The results of the saccharide composition analyses of the reaction products obtained by allowing the enzymes to act on glucose as described above are shown in Figs. 6 to 11. The mannose and fructose contents in the reaction products were calculated on the basis of the peak area ratios observed in the HPLC analysis.

Mannose was formed from glucose in all the test groups of pH 6.0, 7.0, and 8.0 of the solution 1 of the enzyme of the present invention addition groups and the comparative enzyme addition groups. Comparing the mannose contents obtained in the solution 1 of the enzyme of the present invention addition groups and the comparative enzyme addition groups, it was found that the solution 1 of the enzyme of the present invention provided an approximately 1.3 to 2.0 times higher mannose content in all the test groups. On the other hand, no mannose was produced in the test group using the empty vector control at any of the pH levels tested.

Under the conditions that 330.0 µL/g-ds of the solution 1 of the enzyme of the present invention was added, and the reaction solution was kept at a reaction temperature of 50°C for 72 hours, the maximum mannose contents were 28.7% at pH 6.0, 27.2% at pH 7.0, and 29.6% at pH 8.0. These contents are approximately 1.3 times of those obtained with the comparative enzyme under the same conditions.

As for the groups of pH 6.0, almost no fructose was detected with either the solution 1 of the enzyme of the present invention, the comparative enzyme solution, or the empty vector control. On the other hand, as for the test groups of pH 7.0 and 8.0, fructose production was confirmed with all the solution 1 of the enzyme of the present invention, the comparative enzyme solution, and the empty vector control. When the enzyme was allowed to act at pH 7.0 for 72 hours, the fructose content ranged from 1.1 to 1.6% for all the cases using the solution 1 of the enzyme of the present invention, the comparative enzyme solution, and the empty vector control. When the enzyme was allowed to act at pH 8.0 for 72 hours, the fructose content ranged from 3.2 to 7.2% for all the cases using the solution 1 of the enzyme of the present invention, the comparative enzyme solution, and the empty vector control. There was observed a tendency that a higher enzyme addition amount provided a lower fructose content in the reaction products at both pH levels of 7.0 and 8.0.

When the solution 1 of the enzyme of the present invention was added in an amount of 330.0 µL/g-ds and allowed to act at pH 8.0 for 72 hours, the mannose content in the reaction product was 29.6%, the fructose content in the reaction product was 3.4%, and thus the fructose byproduct in the reaction product was equivalent to about 11.4% of mannose. When the solution 1 of the enzyme of the present invention was added in an amount 330.0 µL/g-ds and allowed to act at pH 7.0 for 72 hours, the mannose content in the reaction product was 27.2%, the fructose content in the reaction product was 1.2%, and thus the fructose byproduct in the reaction product was equivalent to about 4.4% of the mannose. On the other hand, when the solution 1 of the enzyme of the present invention was added in an amount of 330.0 µL/g-ds and allowed to act at pH 6.0 for 72 hours, the mannose content in the reaction product was 28.7%, the fructose content in the reaction product was 0.9%, and thus the fructose byproduct in the reaction product was equivalent to about 3.1% of the mannose.

These results suggest that fructose was formed by non-enzymatic reactions (e.g., isomerization of glucose to fructose caused by influence of buffer or pH etc.) or by reactions caused by contaminating enzymes (conversion of glucose to fructose by isomerase). Furthermore, it was also found that the formation of fructose can be suppressed by conducting the reaction at pH 7.0 or lower, especially at pH 6.0.

### Example 5: Talose synthesis test

### 1. Method

A talose synthesis test using galactose as the substrate was conducted with the solution 2 of the enzyme of the present invention. That is, 1 kg of galactose was dissolved in water, then 178.5 g of the solution 2 of the enzyme of the present invention was added to the solution, the pH was adjusted to 6.0, water was added so that the weight of the reaction solution was 2 kg, and these components were mixed. The reaction was allowed in the reaction mixture at 53°C for 24 to 72 hours. Samples of the reaction solution collected at various reaction times were adjusted to pH 4.0 or lower, and boiled for 10 minutes to terminate the reaction. Then, 10 µL of each reaction solution was diluted by mixing it with 800 µL of pure water.

The saccharide composition for talose, tagatose and galactose in the obtained diluted reaction mixture was analyzed by HPLC. The analysis conditions were as follows: column: HILICpak VG-50 4E (Shodex), and HILICpak VG-50G 4A (Shodex); eluent, 80% acetonitrile; flow rate, 0.6 mL/min; column temperature, 40°C; and detector, charged particle detector.

### 2. Results

The results of the saccharide composition analysis for the reaction product obtained by allowing the solution 2 of the enzyme of the present invention to act on galactose are shown in Table 4.

**[Table 4]**

| Reaction time | Saccharide composition of reaction product (%) | | | Total (%) |
|---|---|---|---|---|
| | Talose | Tagatose | Galactose | |
| 0 Hour | 0.0 | 0.0 | 100.0 | 100.0 |
| 24 Hours | 14.7 | 4.8 | 80.5 | 100.0 |
| 48 Hours | 16.9 | 5.6 | 77.6 | 100.0 |
| 72 Hours | 17.5 | 5.9 | 76.5 | 100.0 |
| *Calculated from peak area ratios obtained in HPLC analysis. | | | | |

The enzyme of the present invention can catalyze the reaction at a reaction temperature of 53°C, and produced talose and tagatose from galactose. The maximum talose content was 17.5%.

### Sequence Listing Free Text

SEQ ID NO: 1, Amino acid sequence of the protein derived from *Lunatimonas lonarensis*
SEQ ID NO: 2, Nucleotide sequence encoding the protein derived from *Lunatimonas lonarensis*
SEQ ID NO: 3, Amino acid sequence of the protein derived from *Lunatimonas lonarensis*
SEQ ID NO: 4, Nucleotide sequence encoding the protein derived from *Lunatimonas lonarensis*
SEQ ID NOS: 5 to 8, Primer sequences
SEQ ID NO: 9, Amino acid sequence of the protein derived from *Melioribacter roseus*
SEQ ID NO: 10, Nucleotide sequence encoding the protein derived from *Melioribacter roseus*
SEQ ID NOS: 11 and 12: Primer sequences

## Claims

1. An enzyme preparation for catalyzing an epimerization reaction of a saccharide, which contains any of the proteins (a) to (c) mentioned below, and wherein the saccharide is any selected from the group consisting of glucose, mannose, talose and galactose:
(a) a protein consisting of the amino acid sequence of SEQ ID NO: 1 or 3:
(b) a protein consisting of an amino acid sequence having an amino acid sequence identity of 90% or higher to the amino acid sequence of SEQ ID NO: 1 or 3, and having an activity for catalyzing an epimerization reaction of a saccharide, and
(c) a protein consisting of an amino acid sequence derived from the amino acid sequence of SEQ ID NO: 1 or 3 by substitution, insertion, deletion and/or addition of one or several amino acids, and having an activity for catalyzing an epimerization reaction of a saccharide.

2. An enzyme preparation for catalyzing an epimerization reaction of a saccharide, which contains a culture supernatant of a transformed cell into which any of the polynucleotides (d) to (f) mentioned below has been introduced, and wherein the saccharide is any selected from the group consisting of glucose, mannose, talose and galactose:
(d) a polynucleotide consisting of the nucleotide sequence of SEQ ID NO: 2 or 4;
(e) a polynucleotide that hybridizes with a complementary strand of the polynucleotide consisting of the nucleotide sequence of SEQ ID NO: 2 or 4 under stringent conditions, and encodes a protein having an activity for catalyzing an epimerization reaction of a saccharide; and
(f) a polynucleotide having a nucleotide sequence identity of 90% or higher to the polynucleotide consisting of the nucleotide sequence of SEQ ID NO: 2 or 4, and encoding a protein having an activity for catalyzing an epimerization reaction of a saccharide.

3. A method for producing an epimerization reaction product of a saccharide, which comprises the step of allowing the enzyme preparation according to claim 1 or 2 to act on a saccharide substrate to obtain the epimerization reaction product of a saccharide.

4. A method for producing a food, feed, feed for fish culture, cosmetic, or medicament, which comprises the step of obtaining the food, feed, feed for fish culture, cosmetic, or medicament by using an epimerization reaction product of a saccharide obtained by implementing the method according to claim 3.
